# EUROPEAN PATENT APPLICATION

(11) **EP 1 632 243 A1**
(43) Date of publication of application: **08.03.2006**
(21) Application number: 03735247.3
(22) Date of filing: 09.06.2003
(51) Int. Cl.: A61K 38/10, A61P 31/12, A61P 31/18, C07K 7/08

(54) **USE OF HAB 18G/CD 147 MOLECULE AS TARGET OF ANTIVIRAL ANTAGONIST AND THUS OBTAINED ANTIVIRAL ANTAGONIST**

(30) Priority: 15.05.2003 CN 03108029
(71) Applicant: Chen, Zhi Nan, Xi'an, Shannxi 710032 (CN)
(72) Inventor: Chen, Zhi Nan, Xi'an, Shannxi 710032 (CN)
(74) Representative: Winckels, Johannes Hubertus F.
(86) International application number: PCT/CN2003/000451
(87) International publication number: WO 2004/100974

(57) **Abstract**

The present invention relates to use of HAb 18G/CD 147 molecule as target for designing antiviral antagonist and the antiviral antagonist designed by using such molecule, especially antagonist against SARS Coronavirus and AIDS virus HIV-1. Antagonist of the present invention includes antibody, polypeptide, immune complex, derivative, small molecule compound and so on, which is able to prevent SARS Coronavirus and AIDS virus (HIV-1) from invading host cell, thereby exerts its antagonistic antiviral effect. It is provided by in vitro experiment that such antagonist has inhibitory effect against SARS Coronavirus and AIDS virus (HIV-1), thus is potential antiviral candidate drug.

## Description

### FIELD OF THE INVENTION

The present invention relates to an antiviral medicament designed to target HAb18G/CD147 molecules, in particular to a series of antagonists against SARS Coronavirus and AIDS virus (HIV-1).

### BACKGROUND OF THE INVENTION

Currently Severe Acute Respiratory Syndrome (SARS) is prevalent in almost 33 countries/areas in the world. By June 3, 2003, there were total 8398 SARS patients in the world, among which 772 have died. In mainland China, 5329 SARS patients have been reported in 25 provinces, among which 334 have died.

The SARS pathogen has been proven to be a novel coronavirus, named SARS Coronavirus. Characteristics of SARS include transmission in a short distance, wide spread, rapid onset and rapid progression. SARS patients mainly present fever, dry cough, pneumonic consolidation and respiratory failure. Currently there is no vaccine or special effective medicament available for SARS due to the rapid replication of the virus and the short time since its identification. Today, clinical treatment of SARS includes application of: 1. anti-viral drugs such as neuraminidase inhibitors (oseltamivir), virazole (ribavirin) and thymosin; 2. glucocorticoid (e.g. dexamethasone); 3. Continuous Positive Airway Pressure; 4. IgM; 5. traditional Chinese medicine; etc. However, none of the above has had a noticeable effect.

Acquired Immune Deficiency Syndrome (AIDS) is an important disease that the Chinese government is trying to control and also a major problem, as well as a hot issue in the world, related to public health. The Joint United Nations Programme on HIV/AIDS and the World Health Organization (WHO) published a report on World AIDS Day December 1^{st}, 2002, showing that AIDS is more and more prevalent in the world. For example, HIV infects 15,000 people per day, compared with 8,000 per day before 1995. In the past 20 years, the number of HIV carriers in the world has risen to more than 60 million, among which more than 20 million have died. Specifically, 3.1 million people died of AIDS in 2002. In addition, AIDS prevalence has lowered the average expected life span of some countries in southern Africa from 60-70 to 40-50 years old. The Gross Domestic Product (GDP) of these countries also decreased by 10%.

Until today, no reports about antagonists such as monoclonal antibodies, polypeptides, derivatives, immune complexes, small molecule compounds and preventive medicaments for SARS were available. Neither have there been medicaments such as effective receptor antagonists available for AIDS, except for reverse transcriptase inhibitors. Therefore, it is important and urgent to develop specific anti-SARS virus medicaments and anti-HIV medicaments to decrease the incidence rate and death rate.

The present inventor obtained a monoclonal antibody with high affinity to liver cancer, named HAb18 (Monoclonal Antibodies, .1990; 8(1):11), based on a long-term research of immune-targeting medicine for liver cancer. Using this monoclonal antibody as a ligand, a membrane antigen in the raw extract of a human hepatoma cell line (HHCC) was purified by affinity chromatography and purified natural HAb18G antigen was obtained. The sequence encoding the antigen was subsequently obtained by screening the cDNA library of human liver cancer tissue with the monoclonal antibody. This sequence was identified by GenBank search to be highly homologous to leukocyte differentiation antigen CD 147, so this molecule was determined to be a member of the CD147 family and named HAb18G/CD147 (*Cancer Research on Prevention and Treatment,* 2000;27(1):7-10). Using a basement membrane invasion experiment and gelatin zymography analysis, the inventor demonstrated that the effects of HAb18G molecules in tumors, inflammation and viral infection are interrelated (*Chinese Journal of Clinical Hepatology,* 2002;18(1):55-56; *Cancer Research on Prevention and Treatment,* 2001, 28(4):262-263; *Chinese Journal of Histochemistry and Cytochemistry,* 2001, 10(3):270-272; *Chinese Journal of Cellular and Molecular Immunology,* 1999, 15(1):30-34). More surprisingly, the present inventor discovered that the SARS virus and HIV-1 invade the host through the target system of HAb18G/CD147 receptor. Thus in the present invention, the inventor tried to develop anti-SARS virus drugs and anti-HIV-1 drugs. Some antagonists that have certain inhibitory effects against SARS virus and HIV-1 have thus been obtained.

### BRIEF SUMMARY OF THE INVENTION

According to one aspect of the present invention, use of the HAb18G/CD147 molecule as a target to design anti-viral drugs, in particular anti-SARS virus drugs and anti-HIV-1 drugs, is provided. The amino acid sequence of the HAb18G/CD147 molecule is shown as SEQ ID NO:12.

According to another aspect of the present invention, target antagonists of SARS Coronavirus and HIV-1 that target the HAb18G/CD147 receptor are provided. These antagonists are designed based on the HAb18G/CD147 molecule. Antagonists of the present invention include HAb 18 antibody, peptide antagonists against HAb18G/CD147, or serial correlated antibodies, polypeptides, immune complexes, derivatives and small molecule compounds against HAb18G/CD147.

According to one embodiment of the present invention, the anti-SARS virus drug and the anti-HIV-1 drug is an HAb18 antibody, the gene of the variable region of the light chain of the antibody comprises the sequence of SEQ ID NO:1, and the gene of the variable region of the heavy chain of the antibody comprises the sequence of SEQ ID NO:2.

According to another embodiment of the present invention, the anti-SARS virus drug and anti-HIV-1 drug is an HAb18G/CD147 peptide antagonist selected from a group consisting of: 
Met Thr His Asp Pro Val Ile Ser Leu Pro Thr Thr(SEQ ID NO:3);
Leu His Arg His Ser His Gly His Ser Tyr Lys Ser(SEQ ID NO:4);
Gly His Trp His Asn His Arg His Gln Ala Pro Leu(SEQ ID NO:5);
Lys Tyr Pro His Gln His Leu His Met His Asp Ser(SEQ ID NO:6);
Ile Gly Trp His Tyr Tyr Leu Arg Thr Gln His Ser(SEQ ID NO:7);
Tyr Pro-Phe His His Lys His Typ His Arg Pro Asn(SEQ ID NO:8);
Ala Asn Ile Val Pro Ile His Ala Asn His Phe Gln(SEQ ID NO:9);
Met His Lys His Pro His Gly Ser Gln Gly Pro Thr(SEQ ID NO:10);
 and

Tyr Lys Leu Pro Gly His His His His Tyr Arg Pro(SEQ ID NO:11).

### DETAILED DESCRIPTION OF THE INVENTION

Based on the fact that Human Immunodeficiency Virus (HIV-1) is able to form a complex with Cyclophilin A and on the fact Cyclophilin A facilitates cell entry of the virus through binding to the cell membrane receptor CD 147, and considering SARS Coronavirus enters host cells through the Cyclophilin A-HAb18G/CD147 system as well and thus amplifies, the present inventor designs, screens and synthesizes serial antibodies and peptide antagonists, including e.g. antibodies, polypeptides, complexes, derivatives and small molecule compounds, which target HAb 18G/CD 147. These distinct drug molecules or precursors thereof have the common properties of antagonizing the SARS Coronavirus or AIDS virus by protecting the target site, e.g. the steric structure of the cell surface receptor HAb18G/CD147 which binds to the viruses during infection.

According to the present invention, the antagonists and antibodies include, but are not limited to, HAb18 antibody, peptide antagonists against HAb18G/CD147, or serial correlated antibodies, polypeptides, immune complexes, derivatives and small molecule compounds against HAb18G/CD147. Preferred antagonists of the invention are peptides as set forth in SEQ ID NOs:3-11, the peptides are named respectively HAb18G-AP-1 through HAb18G-AP-9. Physical and chemical properties of nine high-affinity peptide antagonists targeting HAb18G/CD147, which are described as SEQ ID NOs:3-11, are shown in Table 1 as follows:

**Table 1**

| Peptide | SEQ ID NO | Acidic amino acids (%) | Basic amino acids (%) | Hydrophobic amino acids (%) | Molecular weight | pI | Average hydrophobicity index | Half life (h) |
|---|---|---|---|---|---|---|---|---|
| AP-1 | 3 | 8.3 | 8.3 | 41.7 | 1311.5 | 5.06 | 0.133 | 30.0 |

| | | | | | | | | |
|---|---|---|---|---|---|---|---|---|
| AP-2 | 4 | 0.0 | 50.0 | 16.7 | 1445.5 | 9.99 | -1.792 | 5.5 |
| AP-3 | 5 | 0.0 | 41.7 | 30.0 | 1489.6 | 9.78 | -1.800 | 30.0 |
| AP-4 | 6 | 8.3 | 41.7 | 16.7 | 1529.7 | 7.10 | -1.808 | 1.3 |
| AP-5 | 7 | 0.0 | 25.0 | 25.0 | 1560.7 | 8.60 | -0.958 | 20.0 |
| AP-6 | 8 | 0.0 | 50.0 | 25.0 | 1655.8 | 9.99 | -2.275 | 2.8 |
| AP-7 | 9 | 0.0 | 16.7 | 58.3 | 1360.5 | 6.96 | 0.092 | 4.4 |
| AP-8 | 10 | 0.0 | 33.3 | 33.3 | 1313.4 | 8.56 | -1.717 | 30.0 |
| AP-9 | 11 | 0.0 | 50.0 | 33.3 | 1541.7 | 9.70 | -1.967 | 2.8 |

According to the present invention, the antagonists prevent the invasion of SARS Coronavirus, HIV-1 or the like into host cells by occupying the virus receptor site on the host HAb18G/CD147 molecule, thus providing the anti-viral effects. *In vitro* experiments shown in the Examples demonstrated the inhibitory effects of these antagonists against SARS Coronavirus and HIV-1. It is contemplated that other correlated antibodies, polypeptides, immune complexes, derivatives and small molecule compounds against HAb18G/CD147 are included in the present invention as well.

Antagonists described in the present invention can be used in combination with suitable pharmaceutical carriers, thus pharmaceutical compositions comprising antagonists of the present invention are also included in the invention. Pharmaceutical compositions of the invention can be administered transmucosally, subcutaneously, intramuscularly, intraperitoneally, intravenously, intraarterially, or topically.

Genes of the variable regions of the light chain and heavy chain of the described antibody against the SARS coronavirus-HAb18G/CD147 receptor or AIDS virus (HIV-1)-HAb18G/CD147 receptor can be rearranged to generate certain forms of recombinant antibodies (e.g. chimeric antibodies, humanized antibodies, small molecule antibodies, multi-valent micro antibodies, bispecific antibodies, etc.), fusion proteins of recombinant antibodies, antibody-protein immune complexes or derivatives, in order to antagonize the infection of human cells with SARS Coronavirus and AIDS virus.

The serial antagonist peptides against the SARS Coronavirus-HAbl8G/CD147 receptor and AIDS virus (HIV-1)-HAb18G/CD147 receptor can be reconstructed to generate certain forms of polypeptides and complexes thereof, can be derivatized to certain forms of peptides and derivatives thereof, and can be constructed to certain forms of small molecule compounds or complexes, in order to antagonize the infection of human cells with SARS Coronavirus and AIDS virus.

The Examples listed below are for the purpose of describing the present invention more clearly and in more details, but it should be understood that the invention is not limited to these Examples.

### EXAMPLE 1

### Synthesis of Peptide Antagonists and Obtaining Antibodies

### Synthesis of peptide antagonists

The method of solid-phase synthesis using a peptide synthesizer was employed to synthesize the nine peptides with the amino acid sequences of SEQ ID NOs:3-11. Purity of each product was >90%, as analyzed by HPLC (Waters Corporation). The molecular weight was analyzed by MALDI-TOF Mass Spectrometer (Bruker Daltonics Inc.) and the results were in consistence with theoretical scores. Isoelectric Point (pI), hydrophobicity (GRAVY) and *in vivo* half-lives of the peptides were predicted by the ExPASy bioinformatical software package (shown in Table 1).

### Obtaining antibodies and cloning the genes of the variable regions of a monoclonal antibody against HAb18G

The inventor obtained 7 specific monoclonal antibodies against liver cancer by immunizing animals with cell suspensions produced with fresh tumor tissues. Establishment of these antibodies was described in *Monoclonal Antibody Communication,* 1989; 2:33-36. In these antibodies, HAb18 monoclonal antibody had better specificity compared to other strains and had a positive rate of 75% in the immunohistochemistry staining. HAb18 has satisfied the requirements of *The Outline of Preparation of Mouse Morroclonal Antibodies Used in Human and Its Quality Control* published by the National Institute for the Control of Pharmaceutical and Biological Products. Total RNA from HAb18 hybridoma cells was extracted by the guanidinium thiocyanate extraction method and the first strand of cDNA was generated by reverse transcription. The gene of the variable region of the light chain (VL) and the gene of the variable region of the heavy chain (VH) of liver cancer monoclonal antibody HAb18 were successfully amplified with a set of designed universal primers. The intended fragments were subsequently cloned into a T-vector. Sequencing analysis showed their sequences as SEQ ID NOs:1 and 2, respectively.

### EXAMPLE 2

The Example demonstrated the efficacy of anti-SARS Coronavirus peptide antagonists and antibodies obtained in Example 1.

### Pre-experiments:

### 1. Measurement of cytotoxicity of HAb 18G-AP-1, AP-6 and AP-9 to 293 cells

The 293 human embryonic kidney cell line (from the Cell Bank of the Chinese Academy of Sciences) was seeded on a 96-well plate with a concentration of 400,000/ml and incubated at 37°C, under 5% CO₂ for 24 hours. The test drugs were then added. Test drugs HAb18G-AP-1, AP-6 and AP-9 were in a serial dilution of 1000µg/ml to 1.45µg/ml. The positive control drug ganciclovir was in a serial dilution of 6000µg/ml to 11.7µg/ml. Cells at each concentration were seeded in 3 wells, 100µl per well, and normal cells were setup as controls. Cells were incubated at 37°C and under 5% CO₂ for 6 days. A cytopathic effect (CPE) was observed under an inverted microscope everyday. Morphological change of cells under 25% was defined "+", 26%-50% was defined "++", 51%-75% was defined "+++", and 76%-100% was defined "++++". Toxic dose 50 (TD₅₀) and toxic dose 0 (TD₀) of the drugs were calculated by the Reed-Muench method.

### 2. Measurement of the virulence of the No.04 coronavirus in the culture of 293 cells

### Isolation of the No.04 coronavirus:

SARS patients and serum thereof were from Beijing Youan Hospital. 293 cells were seeded in tubes with a concentration of 400,000/ml and incubated at 37°C and under 5% CO₂ for 24 hours. Then the medium was discarded, 0.2ml serum of SARS patient was added in each tube, the tubes were incubated on a rotation drum for 5-7 days. Coronavirus was detected by PCR when CPE appeared. The sample of No.04 exhibited positive results in PCR and was confirmed to be an isolate of coronavirus. The PCR result was still positive after twice purification of the virus with an end point dilution method. Serum of the SARS patient was assayed with immunofluorescence in duplicate. The IgM positive result and a 4-fold risen of IgG concentration confirmed the identification of coronavirus. The virus titer was determined by viral CPE method.

### Viral CPE Method:

293 cells were seeded on a 96-well plate with a concentration of 400,000/ml and incubated at 37°C and under 5% CO₂ for 24 hours. Then the viral solution was added. The virus was diluted to 8 concentrations in a serial dilution from 10⁻¹ to 10⁻⁸. 3 wells were used for each concentration, 100µl per well, and normal cells were setup as control. Cells were incubated at 37°C and under 5% CO₂ for 5-7 days. The cytopathic effect (CPE) was observed under an inverted microscope everyday, morphological change of cells under 25% was defined "+", 26%-50% was defined "++", 51%-75% was defined "+++", and 76%-100% was defined "++++". The Tissue Culture Infective Dose 50 (TCID₅₀) of the virus was calculated by the Reed-Muench method.

### Experiment:

### Inhibitory effects of HAb18G-AP-1, AP-6 and AP-9 on No.04 coronavirus in a 293 cell culture

The purpose of this experiment was to observe the inhibitory effects of HAb18G-AP-1, AP-6 and AP-9 at various concentrations on the No.04 coronavirus in 293 cell culture. The experiment employed the virus infected cell CPE method to calculate the half-inhibitory concentration (IC₅₀), minimum inhibitory concentration (MIC) and treatment index of drugs to thus determine their efficacy.

293 cells were seeded on a 96-well plate with a concentration of 400,000/ml and incubated at 37°C and under 5% CO₂ for 24 hours. Medium was discarded when a cell monolayer formed. Then a solution containing coronavirus at 100 TCID₅₀ was added and normal cells were setup as controls. Cells were incubated at 37°C and under 5% CO₂ for 2 hours. The virus solution was discarded and solutions containing various concentrations of HAb18G-AP-1, AP-6 and AP-9 were added. The drugs were 1:2 diluted into 10 concentrations (i.e. 1000µg/ml to 1.45µg/ml) based on the original concentration of toxic dose 0 (TD₀), while the positive control ganciclovir was 1:2 diluted into 10 concentrations (i.e. 6000µg/ml to 11.7µg/ml). Diluted drugs were added into wells, 3 wells per concentration. Normal cells and virus-infected cells were setup as controls. Cells were incubated at 37°C and under 5% CO₂ for 5-7 days. The cytopathic effect (CPE) was observed under an inverted microscope everyday. The experiments were terminated when a +++ to ++++ morphological change appears in virus-infected cells. The Reed-Muench method was employed to calculate the half-inhibitory concentration (IC₅₀) and minimum inhibitory concentration (MIC) of drugs and treatment index (TI) so as to determine the efficacy. The experiment was performed in triplicate.

### Results:

### Results of pre-experiments:

### Cytotoxicity of HAb18G-AP-1, AP-6 and AP-9 to 293 cells

The above-mentioned drugs and positive control ganciclovir were applied to the 293 cell cultures. The toxic dose 0 (TD₀) and toxic dose 50 (TD₅₀) were calculated using the CPE method. The results listed below were means of triplicate experiments:
1. Results of the test of cytotoxicity of HAb18G-AP-1, AP-6 and AP-9 to 293 cells
   Test drugs:
   HAb18G-AP-1: toxic dose 0 (TD₀): >1000±0µg/ml, toxic dose 50 (TD₅₀): >1000±0µg/ml
   HAb18G-AP-6: toxic dose 0 (TD₀): >1000±0µg/ml, toxic dose 50 (TD₅₀): >1000±0µg/ml
   HAb18G-AP-9: toxic dose 0 (TD₀): >1000±0µg/ml, toxic dose 50 (TD₅₀): >1000±0µg/ml

   Positive control:
   Ganciclovir: toxic dose 0 (TD₀): >6000±0µg/ml, toxic dose 50 (TD₅₀): >6000±0µg/ml
2. Results of the measurement of virulence (TCID₅₀) of the No.04 coronavirus in the culture of 293 cells
   No.04 coronavirus: Tissue Culture Infective Dose 50 (TCID₅₀) is 10⁻³

### Results of experiment:

The inhibitory effects of HAb18G-AP-1, AP-6 and AP-9 on No.04 coronavirus in 293 cell culture (the results listed below were means of triplicate experiments):

### Test drugs:

HAb18G-AP-1: CPE method: half-inhibitory concentration (IC₅₀): 31.25±0µg/ml, minimum inhibitory concentration (MIC): 62.25±0µg/ml, treatment index (TI): 16
HAb 18G-AP-6: CPE method: half-inhibitory concentration (IC₅₀): 31.25±0µg/ml, minimum inhibitory concentration (MIC): 62.25±0µg/ml, treatment index (TI): 16
HAb18G-AP-9: CPE method: half-inhibitory concentration (IC₅₀): 15.6±0µg/ml, minimum inhibitory concentration (MIC): 31.3±0µg/ml, treatment index (TI): 32

### Positive control:

Ganciclovir: CPE method: half-inhibitory concentration (IC₅₀): 11.7±0µg/ml, minimum inhibitory concentration (MIC): 23.44±0µg/ml, treatment index (TI): 256

The experiment above demonstrated that HAb18G-AP-1, AP-6 and AP-9 were capable of inhibiting coronavirus in 293 cell cultures, with the viral CPE method.

### EXAMPLE 3

This example demonstrated the efficacy of anti-HIV peptide antagonists obtained in Example 1.

### 1. Measurement of cytotoxicity of HAb18G-AP-1, AP-6 and AP-9 to MT-4 cells

MT-4 lymphocyte (from Japan) was seeded on a 96-well plate with a concentration of 400,000/ml and incubated at 37°C and under 5% CO₂ for 24 hours. Then test drugs were added. Test drugs HAb18G-AP-1, AP-6 and AP-9 were in a serial dilution of 12000ng/ml to 24ng/ml. Cells at each concentration were seeded to 3 wells, 100µl per well, and normal cells were setup as controls. Cells were incubated at 37°C and under 5% CO₂ for 6 days. Cell growth was subsequently determined by the MTT method.

The results showed that cell growth was not affected and drugs HAb18G-AP-1, AP-6 and AP-9 do not have side effects.

### 2. Inhibitory effects of HAb18G-AP-1, AP-6 and AP-9 on HIV in MT-4 cell cultures

A viral P24 assay was employed to observe the inhibitory effects of HAb18G-AP-1, AP-6 and AP-9 at various concentrations on HIV in MT-4 cell cultures.

Specifically, MT-4 cells were seeded on a 96-well plate with concentrations of 400,000/ml and incubated at 37°C and under 5% CO₂ for 24 hours. The medium was discarded when a cell monolayer was formed. Then a solution containing HIV at 100 TCID₅₀ was added, normal cells and virus infected cells were setup as controls. Cells were incubated at 37°C and under 5% CO₂ for 2 hours. The virus solution was discarded and solutions containing various concentrations of HAb18G-AP-1, AP-6 and AP-9 were added. The drugs were 1:2 diluted into 8 concentrations (i.e. 12000ng/ml~24ng/ml). Diluted drugs were added into wells, 3 wells per concentration. Cells were incubated at 37°C and under 5% CO₂ for 5-7 days prior to analyzing viral protein P24 assay. The experiment was performed in triplicate.

The results of the experiment were shown in Table 2. The results showed that HAb18G-AP-9 is capable of inhibiting HIV-1, while HAb18G-AP-1 and AP-6 are not capable of inhibiting HIV-1.

The experiment above demonstrated that HAb 18G-AP-9 is capable of inhibiting HIV-1, while HAb1BG-AP-1 and AP-6 are not capable of inhibiting HIV-1 in MT-4 cell cultures, with the viral P24 assay.

**Table 2. Results of the HIV P24 assay**

| Concentration of peptide (ng/ml) | 24 | 240 | 480 | 750 | 1500 | 3000 | 6000 | 12000 |
|---|---|---|---|---|---|---|---|---|
| AP-9 | >3 | >3 | >3 | >3 | >3 | 0.265 | 0.396 | 0.298 |
| AP-1 1 | >3 | >3 | >3 | >3 | >3 | >3 | >3 | >3 |
| AP-6 | >3 | >3 | >3 | >3 | >3 | >3 | >3 | >3 |

## Claims

1. Use of HAb18G/CD147 molecule as a target to design anti-viral medicaments, wherein the amino acid sequence of the HAb18G/CD147 molecule is shown as SEQ ID NO:12.

2. The use according to claim 1, wherein the virus is a virus that invades the host through the target system of HAb18G/CD147 molecule receptor.

3. The use according to claim 1, wherein the virus is SARS virus.

4. The use according to claim 1, wherein the virus is HIV-1.

5. A virus antagonist which is an HAb18G/CD147 receptor antagonist of viruses, wherein the antagonist is designed according to the HAb I 8G/CD 147 molecule.

6. An antagonist according to claim 5, which is selected from a group consisting of HAb18 antibody, polypeptide antagonists against HAb 18G/CD 147, or other serial correlated antibodies, polypeptides, immune complexes, derivatives, and small molecule compounds against HAb18G/CD147.

7. A peptide antagonist according to claim 6, wherein the peptide antagonist is an HAb18G/CD147 peptide antagonist selected from a group consisting of: 
Met Thr His Asp Pro Val Ile Ser Leu Pro Thr Thr(SEQ ID NO:3);
Leu His Arg His Ser His Gly His Ser Tyr Lys Ser(SEQ ID NO:4);
Gly His Trp His Asn His Arg His Gln Ala Pro Leu(SEQ ID NO:5);
Lys Tyr Pro His Gln His Leu His Met His Asp Ser(SEQ ID NO:6);
Ile Gly Trp His Tyr Tyr Leu Arg Thr Gln His Ser(SEQ ID NO:7);
Tyr Pro Phe His His Lys His Typ His Arg Pro Asn(SEQ ID NO:8);
Ala Asn Ile Val Pro Ile His Ala Asn His Phe Gln(SEQ ID NO:9);
Met His Lys His Pro His Gly Ser Gln Gly Pro Thr(SEQ ID NO:10);
 and

Tyr Lys Leu Pro Gly His His His His Tyr Arg Pro(SEQ ID NO:11).

8. An antagonist according to any one of claims 5-7, wherein the antagonist is against SARS virus.

9. An antagonist according to any one of claims 5-7, wherein the antagonist is against HIV-1.

10. A pharmaceutical composition, comprising an antagonist according to any one of claims 5-7 and a suitable pharmaceutical carrier.

11. A pharmaceutical composition according to claim 10, wherein the antagonist is one or more antagonist(s) selected from SEQ ID NOs:3-11.


Sequence Listing

<110> CHEN, Zhi Nan

<120> USE OF HAb18G/CD147 MOLECULE AS TARGET FOR ANTIVIRAL ANTAGONISTS
      AND THE ANTIVIRAL ANTAGONISTS THUS OBTAINED

<160> 11

<210> 1
<211> 445
<212> DNA
<213> mouse

<220>
<221> V_region
<222> (14)...(445)

<400> 1
ggggatatcc accatggact cacatactca ggtctttata tttctactgc tctgtgtgtc   60
tggtgcccat gggagtattg tgatgaccca gactcccaca ttcctggttg tatcagcagg  120
agacagggtt accataacct gcaaggccag tcagagtgtg attaatgatg tagcttggta  180
ccaacagaag ccagggcagt ctcctaaact gctgatattc tatgcatcca atcgcaacac  240
tggagttcct gatcgcttca ctggcagtgg atatgggacg gatttcactt tcaccatcag  300
cactgtgcag gctgaagacc tggcagttta tttctgtcag caggattata gtcctccatt  360
cacgttcggc tcggggacaa agctggaaat caaacgcaag tcgactgcac caactgtatc  420
c                                                                  421

<210> 2
<211> 445
<212> DNA
<213> mouse

<220>
<221> V_region
<222> (14)...(408)

<400> 2
ggggatatcc accatgaact tcgggctgag ctgggttttc atagttattc tcttaaaagg   60
tgtccagagt gaagtgaagc ttgaggagtc tggaggaggc ttggtgcaac ctggaggatc  120
catgaaactg tcttgtgttg cctctggatt cacttttagt gacgcctgga tggactgggt  180
ccgccagtct ccagagaagg gacttgagtg ggttgctgaa attagaagca aagctaataa  240
tcatgcacca tactatactg agtctgtgaa agggaggttc accatctcac gagatgattc  300
caaaagtatt atctacctgc aaatgaacaa cttaagagct gaagacactg gcatttatta  360
ctgtaccagg gatagcacgg ctacccactg gggccaaggg actctggtca ctgtctcttc  420
agctagcacg acagccccat cagtc                                        445

<210> 3
<211> 12
<212> PRT
<213> Artificial sequence

<400>3
Met Thr His Asp Pro Val Ile Ser Leu Pro Thr Thr
 1               5                  10

<210> 4
<211> 12
<212> PRT
<213> Artificial sequence

<400> 4
Leu His Arg His Ser His Gly His Ser Tyr Lys Ser
1               5                   10

<210> 5
<211> 12
<212> PRT
<213> Artificial sequence

<400> 5
Gly His Trp His Asn His Arg His Gln Ala Pro Leu
1               5                   10

<210> 6
<211> 12
<212> PRT
<213> Artificial sequence

<400> 6
Lys Tyr Pro His Gln His Leu His Met His Asp Ser
1               5                   10

<210> 7
<211> 12
<212> PRT
<213> Artificial sequence

<400> 7
Ile Gly Trp His Tyr Tyr Leu Arg Thr Gln His Ser
1               5                   10

<210> 8
<211> 12
<212> PRT
<213> Artificial sequence

<400> 8
Tyr Pro Phe His His Lys His Trp His Arg Pro Asn
1               5                   10

<210> 9
<211> 12
<212> PRT
<213> Artificial sequence

<400> 9
Ala Asn Ile Val Pro Ile His Ala Asn His Phe Gln
1               5                   10

<210> 10
<211> 12
<212> PRT
<213> Artificial sequence

<400> 10
Met His Lys His Pro His Gly Ser Gln Gly Pro Thr
1               5                   10

<210> 11
<211> 12
<212> PRT
<213> Artificial sequence

<400> 11
Tyr Lys Leu Pro Gly His His His His Tyr Arg Pro
1               5                   10

<210> 12
<211> 269
<212> PRT
<213> human

<400> 12
Met Ala Ala Ala leu Phe Val Leu Leu Gly Phe Ala leu Leu Gly Thr
1               5                   10                  15

His Gly Ala Ser Gly Ala Ala Gly Thr Val Phe Thr Thr Val Glu Asp
            20                  25                  30

Leu Gly Ser Lys Ile leu leu Thr Cys Ser Leu Asn Asp Ser Ala Thr
        35                  40                  45

Glu Val Thr gly His Arg Trp Leu Lys Gly Gly Val Val Leu Lys Glu
    50                  55                  60

Asp Ala Leu Pro Gly Gln Lys Thr Glu Phe Lys Val Asp Ser Asp Asp
65                  70                  75                  80

Gln Trp Gly Glu Tyr Ser Cys Val Phe Leu Pro Glu Pro Met Gly Thr
                85                  90                  95

Ala Asn Ile Gln Leu His Gly Pro Pro Arg Val Lys Ala Val Lys Ser
            100                 105                 110

Ser Glu His Ile Asn Glu Gly Glu Thr Ala Met Leu Val Cys Lys Ser
        115                 120                 125

Glu Ser Val Pro Pro Val Thr Asp Trp Ala Trp Tyr Lys Ile Thr Asp
    130                 135                 140

Ser Glu Asp Lys Ala Leu Met Asn Gly Ser Glu Ser Arg Phe Phe Val
145                 150                 155                 160

Ser Ser Ser Gln Gly Arg Ser Glu Leu His Ile Glu Asn Leu Asn Met
                165                 170                 175

Glu Ala Asp Pro Gly Gln Tyr Arg Cys Asn Gly Thr Ser Ser Lys Gly
            180                 185                 190

Ser Asp Gln Ala Ile Ile Thr Leu Arg Val Arg Thr His leu Ala Ala
        195                 200                 205

Leu Trp Pro Phe Leu Gly Ile Val Ala Glu Val Leu Val Leu Val Thr
    210                 215                 220

Ile Ile Phe Ile Tyr Glu Lys Arg Arg Lys Pro Glu Asp Val Leu Asp
225                 230                 235                 240

Asp Asp Asp Ala Gly Ser Ala Pro Leu Lys Ser Ser Gly Gln His Gln
                245                 250                 255

Asn Asp Lys Gly Lys Asn Val Arg Gln Arg Asn Ser Ser
            260                 265